# EUROPÄISCHE PATENTSCHRIFT

(11) **EP 1 838 372 B1**
(45) Veröffentlichungstag und Bekanntmachung des Hinweises auf die Patenterteilung: **28.09.2011**
(21) Anmeldenummer: 05787298.8
(22) Anmeldetag: 01.09.2005
(51) Int. Cl.: A61M 16/06

(54) **ATEMMASKE**
RESPIRATORY MASK
MASQUE RESPIRATOIRE

(30) Priorität: 03.09.2004 DE 102004043208
(43) Veröffentlichungstag der Anmeldung: 03.10.2007
(60) Teilanmeldung: 11006459.9
(73) Patentinhaber: Weinmann Geräte für Medizin GmbH & Co. KG, 22525 Hamburg (DE)
(72) Erfinder: SCHULZ, Gerd, 22869 Schenefeld (DE); EIFLER, Martin, 25348 Glückstadt (DE)
(74) Vertreter: Klickow, Hans-Henning
(86) Internationale Anmeldenummer: PCT/DE2005/001535
(87) Internationale Veröffentlichungsnummer: WO 2006/024288

(56) Entgegenhaltungen:
- DE-U1- 29 723 101
- US-A- 5 765 553
- US-A- 5 921 239
- US-A1- 2003 075 180

## Beschreibung

Die Erfindung betrifft eine Atemmaske mit einem Maskenkörper und einem gelenkigen Anschlußstück, welches mit einem Atemschlauch verbindbar ist sowie bei der im Bereich des Maskenkörpers mindestens ein Ausatemspalt angeordnet ist.

Atemmasken werden beispielsweise im Zusammenhang mit Beatmungsgeräten verwendet, um Atemgas zum Patienten zu leiten und eine Ableitung des ausgeatmeten Atemgases zu unterstützen. Die Atemmaske ist typischerweise über den Atemschlauch mit dem Beatmungsgerät verbunden.

Als Nachteil bei den bereits bekannten Atemmasken erweist es sich, daß beim Ausatmen durch den Maskenkörper und den Beatmungsschlauch mit einer für den Patienten und die Umgebung unangenehmen akustischen Beeinträchtigung zu rechnen ist und darüber hinaus der ausgeatmete Luftstrom einen am Patienten entlang streichenden kühlen Luftzug hervorruft.

In der US-A 5,921,239 wird eine Atemmaske mit einem Maskenkörper und einem gelenkigen Anschlußstück beschrieben, das mit einem Atemschlauch verbindbar ist. Im Bereich des Maskenkörpers ist eine Ausströmöffnung für verbrauchtes Atemgas vorgesehen.

Aufgabe der vorliegenden Erfindung ist es, eine komfortable Gestaltung einer Atemmaske bereitzustellen, bei der die Beeinträchtigung des Patienten durch ausgeatmete Luft weitgehend vermieden wird.

Diese Aufgabe wird erfindungsgemäß dadurch gelöst, daß für die Verbindung von Maskenkörper und Anschlußstück eine mechanische Kodierung vorgesehen ist.

Die erfindungsgemäße Atemmaske, die nicht nur als Einzelteil sondern auch als Element eines ganzen Beatmungsgeräts zu verstehen ist, umfaßt einen Maskenkörper sowie ein gelenkiges Anschlußstück, welches mit einem Atemschlauch verbindbar ist, wobei im Bereich des Übergangs zwischen dem gelenkigen Anschlußstück und einem das gelenkige Anschlußstück aufnehmenden Anschluß am Maskenkörper mindestens ein Ausatemspalt angeordnet ist. Insbesondere die Anordnung des Ausatemspalts zwischen dem Maskenkörper und dem Anschlußstück hält den Geräuschpegel gegenüber anderen Anordnungspositionen gering. Auch die CO₂ - Auswaschung wird in dieser Position des Ausatemspalts besonders effektiv durchgeführt. Des weiteren ist es gerade im Übergangsbereich zwischen Maskenkörper und Anschlußstück möglich, eine große Spaltlänge vorzusehen, die schalltechnisch besonders vorteilhaft ist im vergleich beispielsweise zu Löchern und kurzen Schlitzen.

Die Verbindung von Maskenkörper und Anschlußstück erfolgt mit einer mechanischen Kodierung. Das hat zum Vorteil, daß eine fehlerhafte Fixierung von Maskenkörper und Anschlußstück zueinander sowie eine fehlerhafte Kombination eines Anschlußstücks mit einem Maskenkörper verhindert werden.

Der mindestens eine Ausatemspalt wird bevorzugt von zwei Ausströmflächen begrenzt.

Das gelenkige Anschlußstück ist vorteilhafterweise als Kugelgelenk ausgebildet und liegt an einzelnen Punkten, insbesondere an zwei Punkten, in einem Kugelkäfig des aufnehmenden Anschlusses auf. Auf diese Weise tritt eine geringe Lagerreibung auf und eine leichte Beweglichkeit des angeschlossenen Atemschlauchs ist gewährleistet. Ebenfalls können in einfacher Weise Toleranzen ausgeglichen werden.

In einer weiteren vorteilhaften Ausgestaltung der Atemmaske wird der mindestens eine Ausatemspalt von den Ausströmflächen begrenzt, wobei diese sich benachbart zu mindestens einem Distanzelement befinden und dabei spielfrei oder sogar mit Vorspannung zueinander versehen sind. So wird vermieden, daß durch entweichende Luft bei bestehendem Spiel unerwünschte Schwingungen und Resonanzen auftreten. Insbesondere bei einem Ausatemspalt in einem Kugelgelenk ergibt sich nämlich eine große Spaltlänge. Dies könnte sich unter Umständen als schalltechnisch nachteilhaft erweisen, wenn sich im Spalt bewegliche Teile befinden.

Der Ausatemspalt zwischen zwei Ausströmflächen kann zudem weitgehend toleranzfrei hergestellt werden, wenn die spaltbildenden Flächen durch eine Vorspannung aneinandergedrückt werden und hierdurch kein Spiel aufweisen, und die Spalthöhe durch Distanzelemente zwischen den Flächen eingestellt wird. Bei einer solchen Konstruktion hängt die Spalthöhe abgesehen von Form- und Lagetoleranzen der spaltbildenden Flächen nur von der Toleranz einer Rippenhöhe als Distanzelement ab. Da typische Rippenhöhen in Ausatemspalten zwischen 0,1 und 0,5 mm liegen, sind Spalttoleranzen von +- 0,005 mm und feiner prozeßsicher herstellbar. Flow- und Schallemissionen werden folglich in sehr engen Grenzen gehalten.

Die Fixierung von Maskenkörper und Anschlußstück zueinander erfolgt vorteilhaft über einen Sicherungsring mit einer Schließeinrichtung, beispielsweise mit einem Bajonettverschluß. Durch den Sicherungsring werden auch die Ausatemspalte fixiert, indem zum Beispiel die Ausströmflächen der Atemmaske und komplementäre Ausströmflächen am Sicherungsring gegeneinander vorgespannt werden.

Eine zusätzliche vorteilhafte Ausgestaltung der Atemmaske besteht darin, daß die Ausströmflächen einen Ausströmkanal bilden, der der Atemgasstrom in einem Winkel zwischen 10° und 45°, insbesondere zwischen 20° und 30°, besonders bevorzugt von etwa 25°, bezogen auf eine Ebene frontal zum Gesicht eines Patienten, ableitet. Auf diese Weise wird die ausgeatmete Luft im wesentlichen schirmförmig vom Patienten weggeleitet und macht sich für den Patienten nicht unangenehm bemerkbar.

Des weiteren kann der Abströmkanal so ausgebildet sein, daß er ein Ableiten der Ausatemströmung in Richtung der Augenpartie des Patienten ausschließt, da sich ein Luftzug an den Augen als besonders unangenehm erweist.

Weiterhin kann die Atemmaske so ausgebildet sein, daß an den Ausströmungskanal grenzende Oberflächen der Atemmaske aus hartem Kunststoff gefertigt sind und Oberflächen von mit der Hand bedienbaren Elementen der Atemmaske aus weichem Kunststoff gefertigt sind. Auf diese Weise wird einerseits der Atemstrom zuverlässig abgeleitet und andererseits die Handhabbarkeit der Atemmaske erleichtert.

Die Erfindung wird im folgenden beispielhaft anhand der Figuren erläutert. Es zeigen:
- Fig. 1: eine als Nasalmaske ausgebildete Atemmaske in perspektivischer Ansicht,
- Fig. 2: eine Ansicht auf das Innere der Maske aus Fig. 1 von hinten,
- Fig. 3: eine Ansicht auf das Äußere der Maske aus Fig. 1 von vorne,
- Fig. 4: den Grundkörper der Maske aus Fig. 1 in per- spektivischer Ansicht,
- Fig. 5: eine Seitenansicht des Grundkörpers,
- Fig. 6: einen Querschnitt durch die Symmetrieebene des Grundkörpers,
- Fig. 7: eine Ansicht auf den Sicherungsring von hin- ten,
- Fig. 8: eine Ansicht auf den Sicherungsring von vor- ne,
- Fig. 9: den Sicherungsring in perspektivischer An- sicht,
- Fig. 10: den Sicherungsring in seitlicher Ansicht,
- Fig. 11: den Sicherungsring aus Fig. 10 im Quer- schnitt,
- Fig. 12: eine perspektivische Darstellung des Masken- grundkörpers,
- Fig. 13: eine teilweise Darstellung eines vergrößerten Schnittes durch den Maskengrundkörper im Be- reich des Kugelgelenkes und
- Fig. 14: eine vergrößerte Darstellung der Einzelheit XIV in Fig. 13.

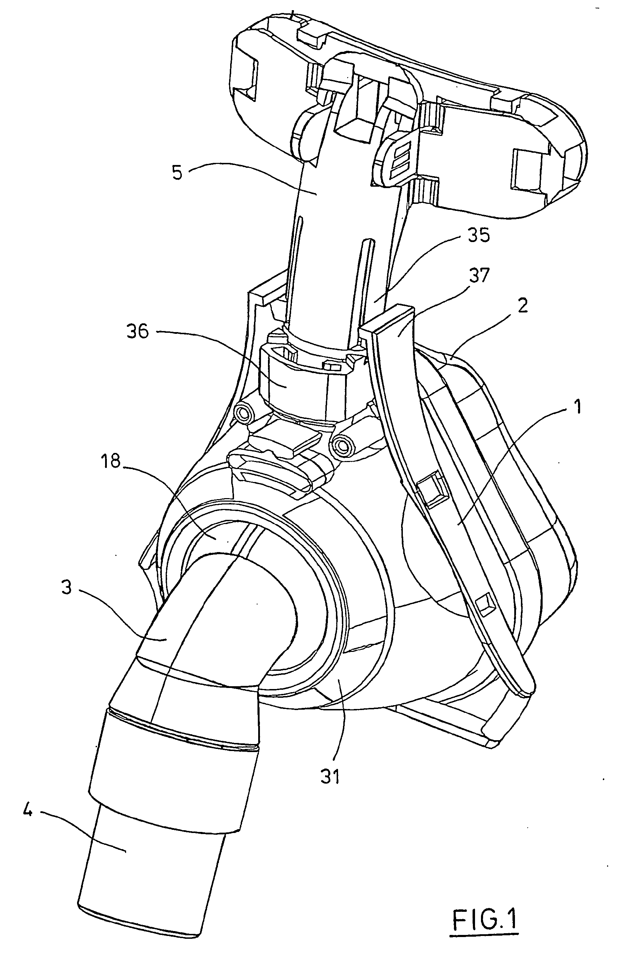

Fig. 1 zeigt eine als Nasalmaske ausgebildete Atemmaske, deren Maskenkörper (1) aus einem relativ festen Material gefertigt ist und die einen Maskenwulst (2) aufweist. Der Maskenwulst (2) dient zur Anlage am Gesicht eines nicht abgebildeten Patienten und gewährleistet die erforderliche Abdichtung. Über ein winkelförmig ausgebildetes Anschlußstück (3) ist der Maskenkörper (1) mit einer drehbeweglich gelagerten Hülse (4) verbunden, die zum Anschluß an einen nicht abgebildeten Atemgasschlauch dient. Zur Gewährleistung einer sicheren Positionierung der Atemmaske im Kopfbereich eines Patienten wird eine Stirnstütze (5) verwendet. Das Anschlußstück (3) und der Maskenkörper (1) sind über ein Kugelgelenk (18) miteinander verbunden.

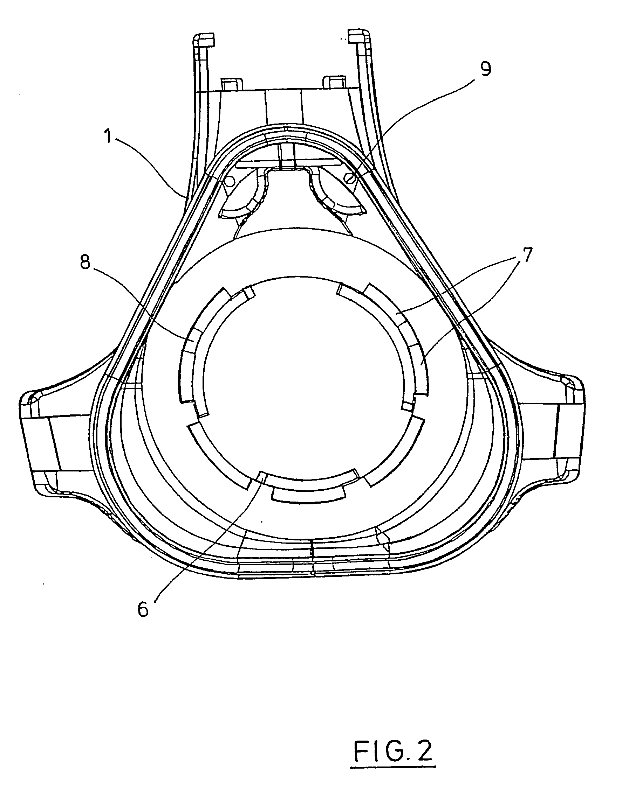

In Fig. 2 ist das Innere der Nasalmaske aus Fig. 1 bei einer Blickrichtung von innen in Richtung auf eine Aufnahme für das hier nicht abgebildete Kugelgelenk (18) dargestellt. Im oberen Bereich sind zwei Druckmeßstutzen (9) angeordnet. Strömungsöffnungen (7) führen zu nicht abgebildeten Ausatemspalten (14). Die Strömungsöffnungen (7) sind von Strömungsleitstrukturen (8) begrenzt. Die Strömungsleitstrukturen (8) sind derart ausgebildet, dass der Atemgasstrom gesammelt und in die Ausatemspalten (14) geleitet wird. Die Strömungsleitstrukturen (8) sind bevorzugt derart ausgebildet, dass der Atemgasstrom trichterförmig in die Ausatemspalten (14) geleitet wird. Die Strömungsleitstruktur (8) im oberen Bereich verschließt den Atemgasstrom in Richtung der Augen eines Anwenders. Einführschrägen (6) sind für das erleichterte Einführen von Bajonettzähnen (26) eines nicht dargestellten Kugelkäfigs (24) vorgesehen.

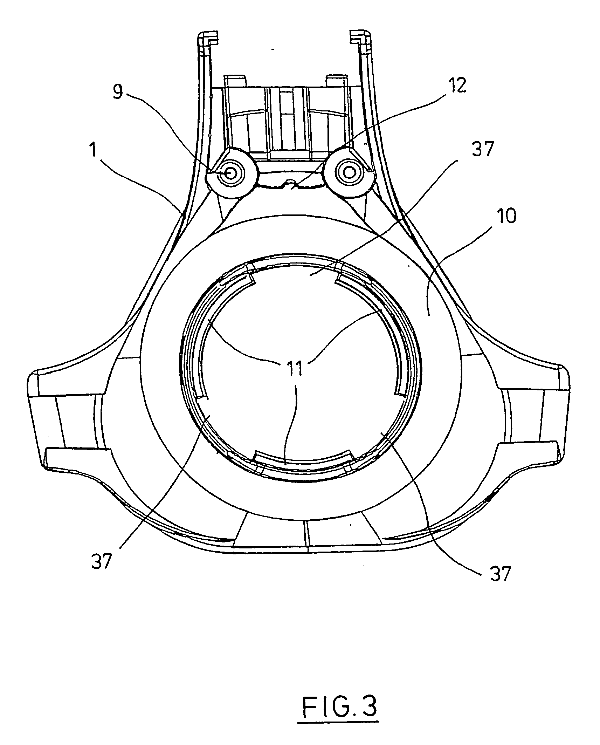

Fig. 3 gibt den Blick auf das Äußere der Maske aus Fig. 1 bei einer Blickrichtung von vorne frei. Eine Ausströmfläche (10) ist ringförmig ausgebildet. Mit Hilfe von Rippen (11) und einer Verrastung (12) kann ein hier nicht abgebildeter Sicherungsring (31) angebracht und fixiert werden.

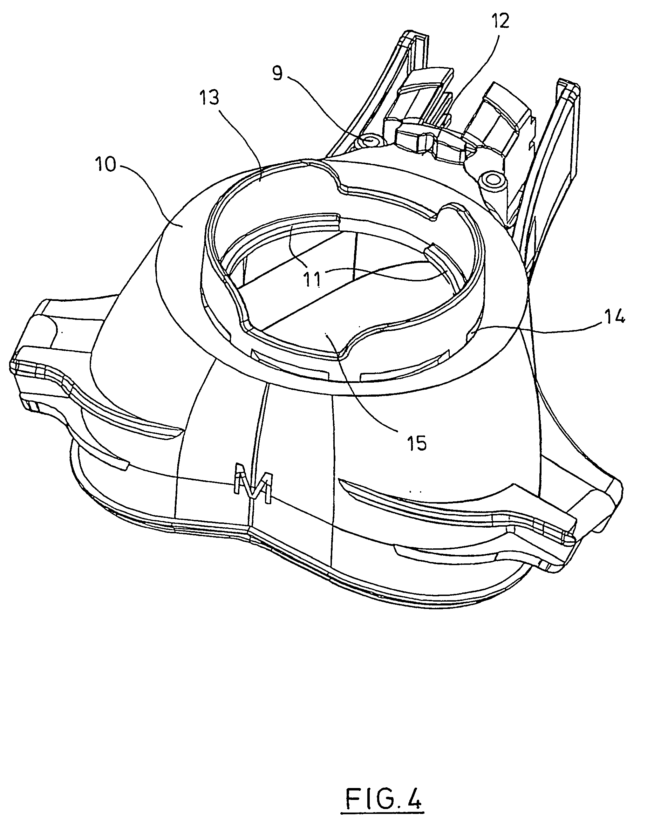

In Fig. 4 ist der Grundkörper der Nasalmaske in perspektivischer Ansicht dargestellt. Ein zentrierring (13) ist für das Aufsetzen des Sicherungsrings vorgesehen, Ausatemspalte (14) befinden sich an den Seiten des Zentrierrings (13) und öffnen sich zur Ausströmfläche (10) hin. Der Zentrierring (13) weist Aussparungen (15) auf, die eine Fehlmontage des Sicherungsrings verhindern, indem sie eine mechanische Kodierung bilden.

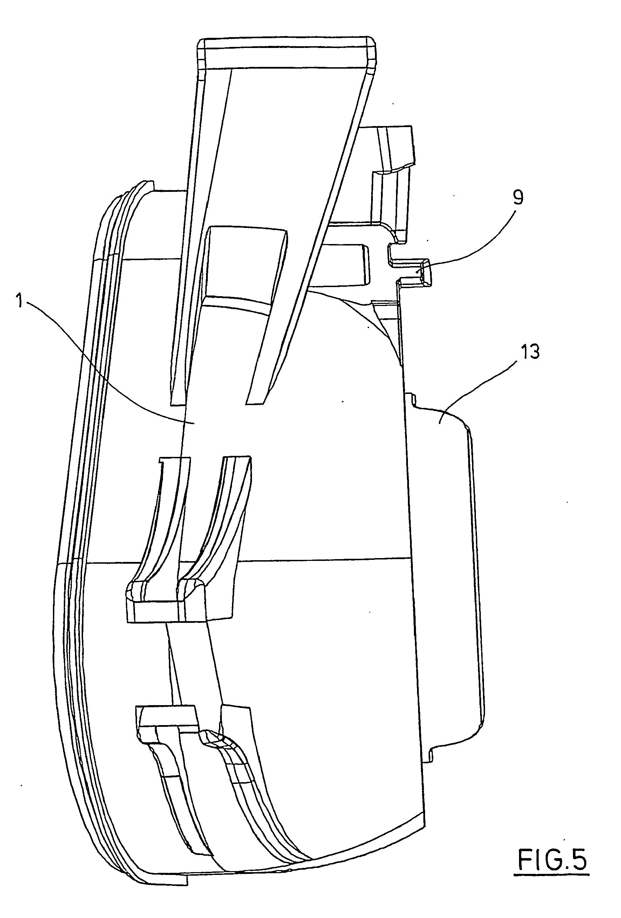

In Fig. 5 ist eine Seitenansicht des Grundkörpers dargestellt. Zu erkennen sind insbesondere der Maskenkörper (1), der zentrierung (13) und die verrastung (12).

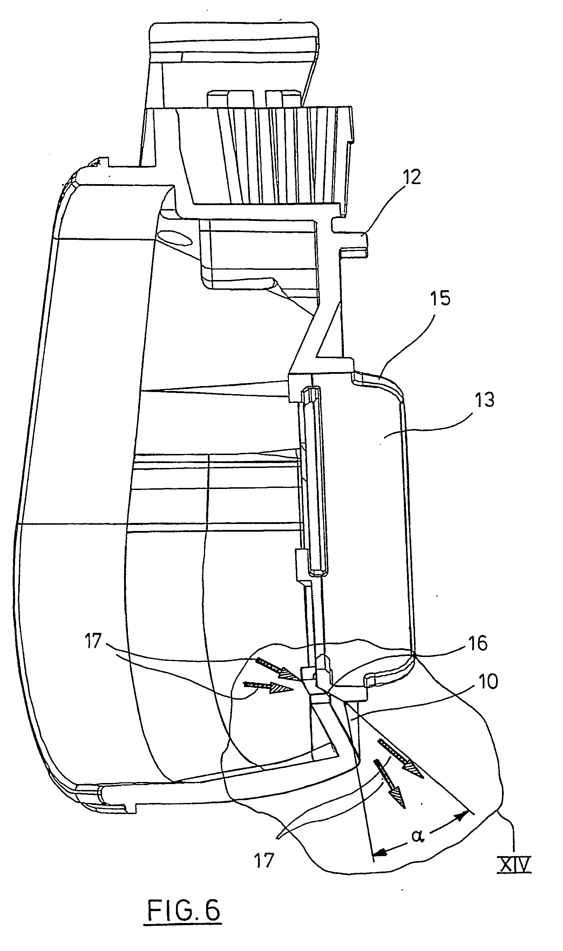

In der Fig. 6 ist der Grundkörper aus Fig. 5 im Querschnitt dargestellt. Der Atemgasstrom (17) gelangt entlang der Strömungsleitstruktur (8) in den Ausatemspalt (14). Die Strömungsleitstruktur (8) ist bevorzugt derart ausgebildet, dass der Atemgasstrom trichterförmig in den Ausatemspalt (14) geleitet wird. Der Ausatemspalt ist durch die Strömungsleitfläche (16) begrenzt. Im Ausatemspalt wird der Atemgasstrom zumindest einmal an der Strömungsleitfläche (16) umgelenkt. Es kann eine Umlenkung der Atemgasströmung nur im Ausatemspalt (14), nur im Bereich der Überleitung des Maskengrundkörpers (1) in den Ausatemspalt (14) oder kombiniert in beiden Bereichen erfolgen.

Entlang der Strömungsleitfläche (16) gelangt der Atemgasstrom vom Inneren der Atemmaske nach außen. Nach Verlassen des Ausatemspaltes (14), der die engste Stelle darstellt, verlässt der Atemgasstrom fächerförmig die Atemmaske entlang der langgestreckten Ausströmfläche (10). Dabei verläßt der Atemgasstrom den Ausatemspalt (14) in einem Winkel Alpha, der vorzugsweise zwischen 10° und 45° zur Vertikalen der Fig. 6 liegt. Diese vertikale Ebene fällt zusammen mit einer Ebene, die frontal zum Gesicht eines hier nicht dargestellten Patienten verläuft.

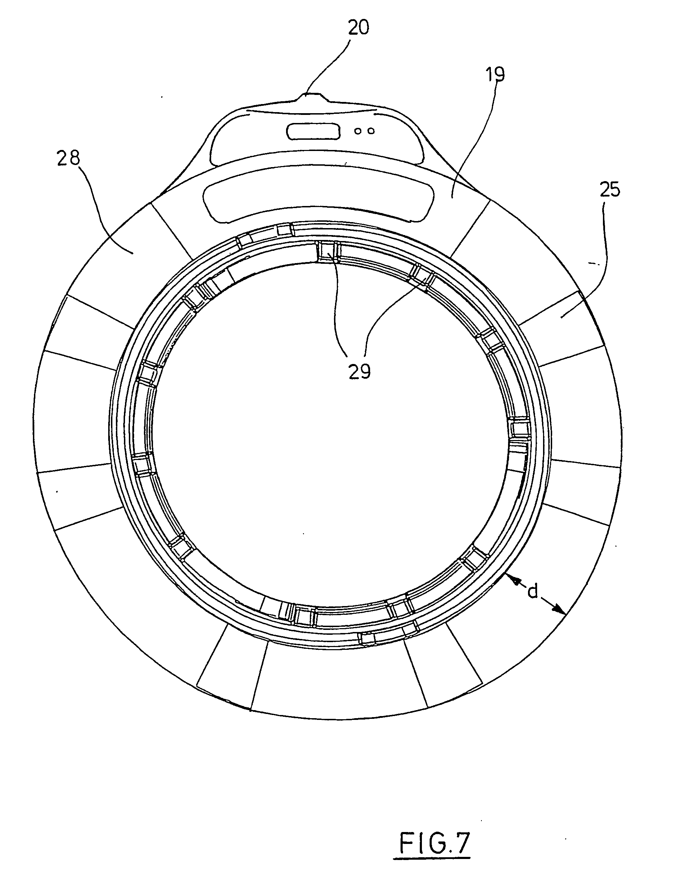

In Fig. 7 ist eine Draufsicht auf einen Sicherungsring (31) von hinten gezeigt, der eine Breite d von weniger als 7 mm aufweist, dargestellt durch einen Doppelpfeil. Distanzrippen (25) ermöglichen eine Montage des Sicherungsrings (31) auf dem Maskenkörper (1) im Bereich der Ausströmfläche (10) ohne Spiel bzw. mit Vorspannung. Ausströmflächen (28) am Sicherungsring (31) begrenzen im montierten Zustand mit der Ausströmfläche (10) am Maskenkörper (1) die Ausatemspalte (14). Eine Nase (20) am Sicherungsring (31) bildet das Komplement zur Verrastung (12) am Maskenkörper (1), so daß eine zusätzliche Befestigung des Sicherungsrings (31) am Maskenkörper (1) gewährleistet wird. Ein in montiertem Zustand an der Ausströmfläche (10) aufliegendes Flächensegment (19) gewährleistet, daß keine Luft in Richtung der Augen der Patienten abströmt. Schlitze (29) zwischen den Elementen des Kugelkäfigs (24) sorgen für eine leichte Montage.

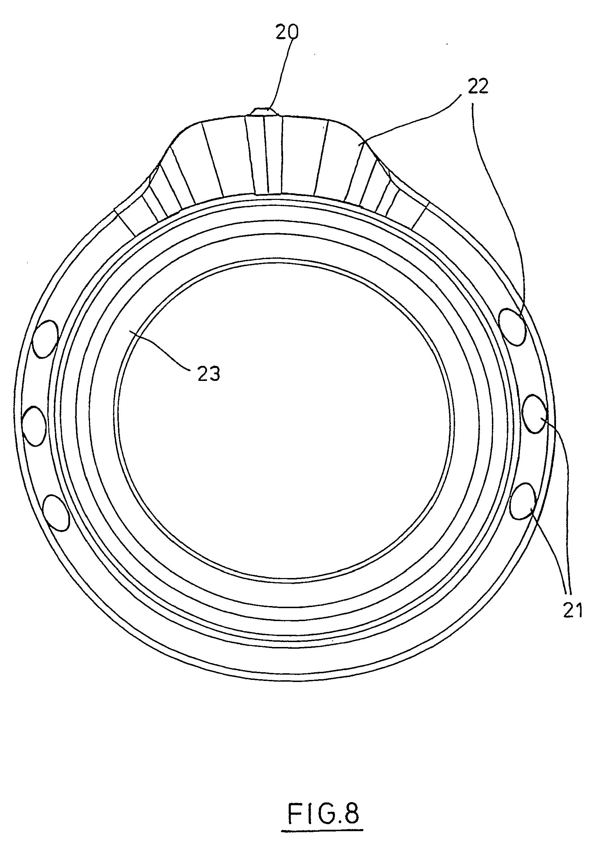

Die Fig. 8 zeigt eine Ansicht auf den Sicherungsring (31) von vorne. Der innere Bereich des Sicherungsrings (31) ist aus Hartmaterial (23) gefertigt, und der äußere Bereich aus einem Weichmaterial (22). Noppen (21) verbessern die Griffigkeit.

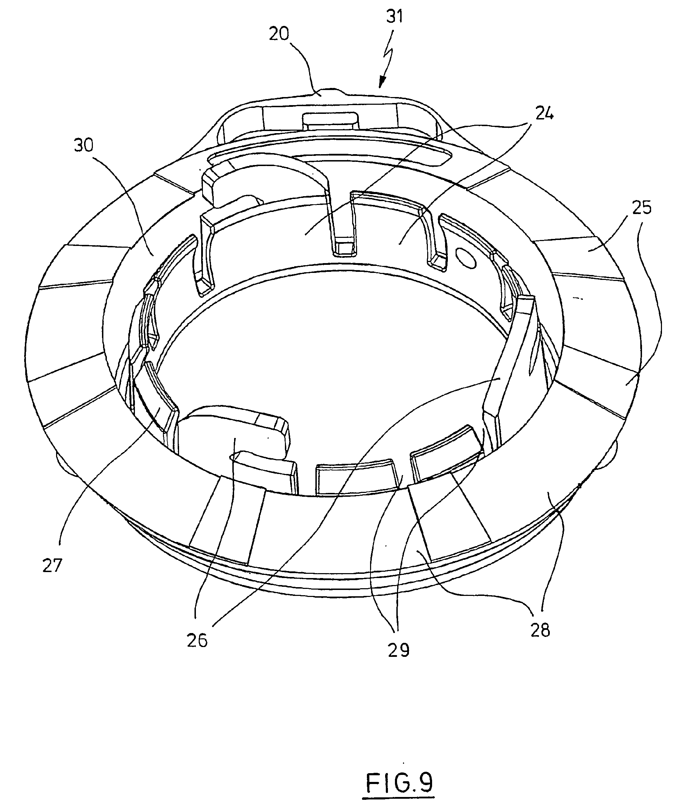

Die Fig. 9 stellt den Sicherungsring (31) in perspektivischer Ansicht von hinten dar. Diese Blickrichtung gewährleistet eine Sicht auf die Aufnahme (30) für den Steg des Zentrierrings (13).

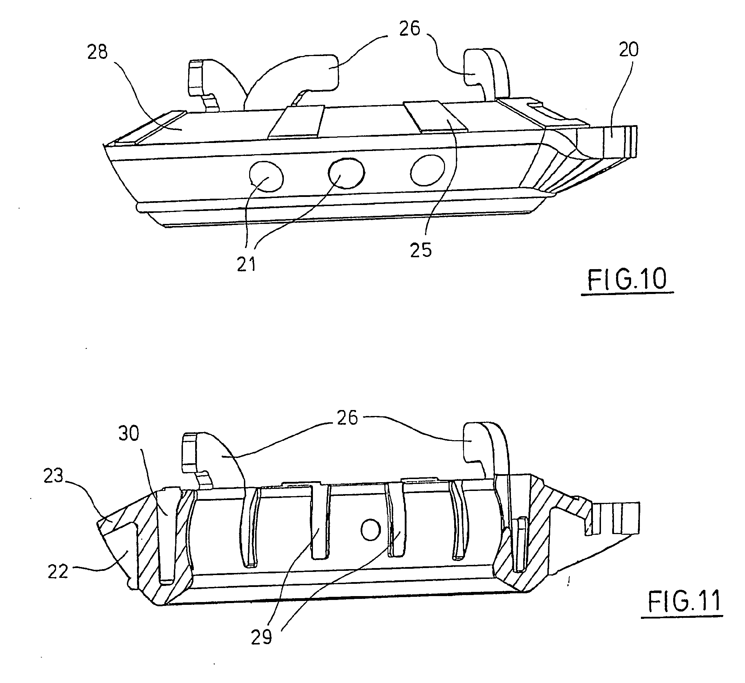

Die Fig. 10 zeigt den Sicherungsring (31) in seitlicher Ansicht und gewährt den Blick auf Bajonettzähne (26).

Die Fig. 11 gibt den Sicherungsring (31) aus Fig. 10 im Querschnitt wieder.

Die Funktionalität der einzelnen in Fig. 9 bis Fig. 11 dargestellten Bauelemente wird später nochmals ausführlich im Zusammenhang mit einer Beschreibung einer Montage und Demontage der einzelnen Bauelemente erläutert, um insbesondere die mechanische Bedeutung und Funktionalität der einzelnen Bauelemente weiter zu veranschaulichen.

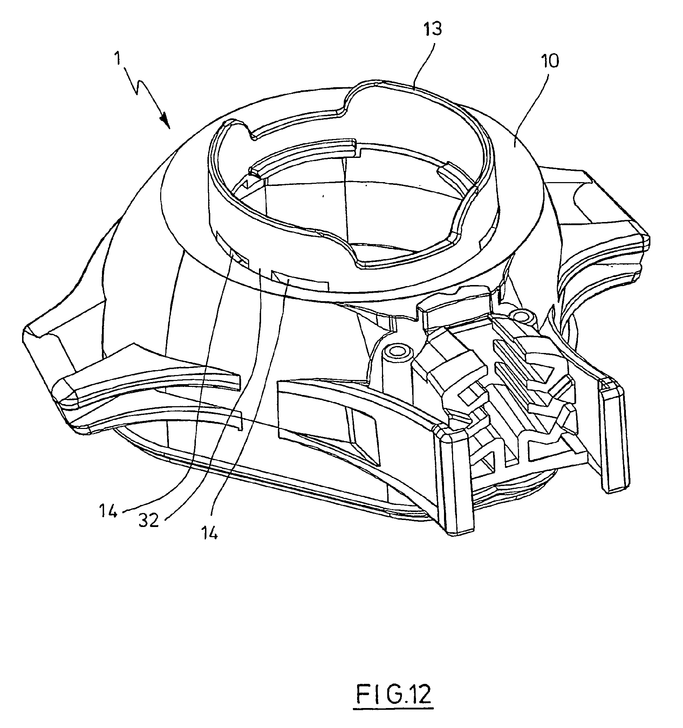

Die perspektivische Darstellung in Fig. 12 veranschaulicht nochmals den Maskenkörper (1) nach einem Abnehmen der in Fig. 12 nicht dargestellten Stirnstütze (5). Im Übergangsbereich zwischen der Ausströmfläche (10) und dem zentrierring (13) ist insbesondere noch einmal die Anordnung der Ausatemspalte (14) zu erkennen. Die Ausatemspalte (14) besitzen im wesentlichen rechteckförmige Querschnittgestaltungen und verlaufen mit ihren Längsachsen in einer Umfangsrichtung der Ausströmfläche (10). Die einzelnen Ausatemspalte (14) sind von Distanzelementen (32) voneinander getrennt. Die Distanzelemente (32) führen zu einer mechanischen Verbindung zwischen dem Zentrierring (13) und dem weiteren Material des Maskenkörpers (1).

Die Ausatemspalte (14) sind vorzugsweise derart angeordnet, daß sie in einem der Ausströmfläche (10) zugewandten Bereich des Zentrierringes (13) verlaufen. Hierdurch wird das aus den Ausatemspalten (14) austretende Atemgas unmittelbar in den Bereich der Ausströmfläche (10) geleitet. Nach Verlassen der Ausatemspalten (14) wird der Atemgasstrom an den langgestreckten und breiten Ausströmflächen (10) (28) umgelenkt und strömt diffus und leise, durch den von den Ausströmflächen definierten Ausströmkanal, in die Umgebung ab.

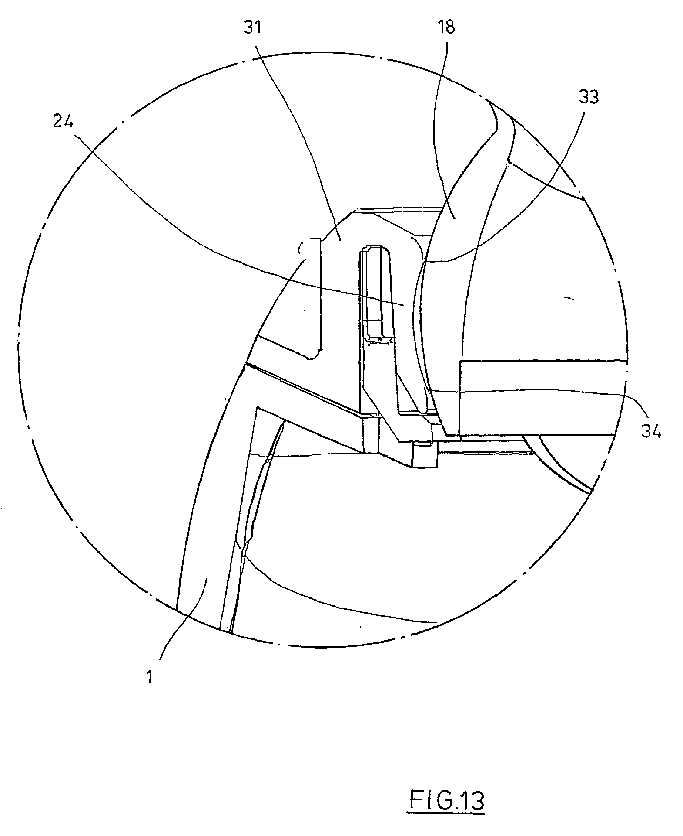

Fig. 13 zeigt eine vergrößerte teilweise Schnittdarstellung im Übergangsbereich zwischen dem Kugelgelenk (18) und dem vom Maskenkörper (1) gehalterten Sicherungsring (31). Zu erkennen ist insbesondere ein Wölbungsverlauf des Kugelkäfigs (24). Der Kugelkäfig (24) verläuft hierbei konkav mit einer stärkeren Krümmung als eine konvex ausgebildete äußere Oberfläche des Kugelgelenkes (18). Das Kugelgelenk (18) wird hierdurch nur entlang zweier Führungslinien, Führungspunkte (33, 34) oder entlang von Liniensegmenten vom Kugelkäfig (24) beaufschlagt. Hierdurch können eventuell im Bereich der Oberflächen des Kugelgelenkes (18) und/oder des Kugelkäfigs (24) vorliegende Fertigungstoleranzen ausgeglichen werden, da keine flächige Führung der Bauteile aneinander vorgesehen ist. Vorzugsweise erfolgt eine federnde Verspannung des Kugelgelenkes (18) innerhalb des Kugelkäfigs (24), damit ein gesichertes Aufliegen entlang der Führungslinien (33, 34) sichergestellt ist.

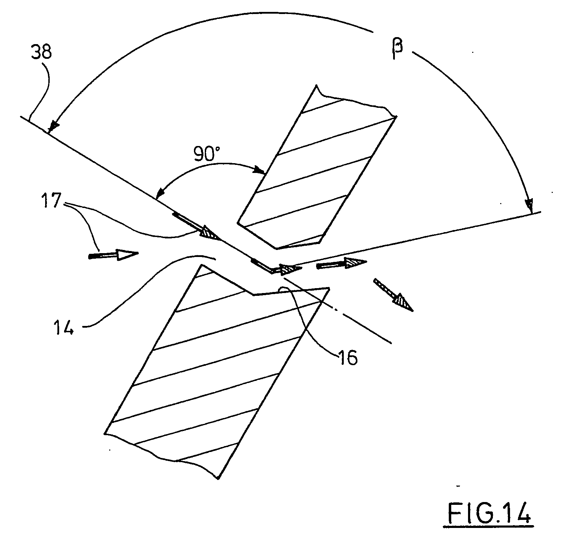

Fig. 14 zeigt einen vergrößerten Ausschnitt der Passage des Atemgasstromes durch den Ausatemspalt (14) entlang der Strömungsleitfläche (16) in die Umgebung (siehe Fig. 6). Der Atemgasstrom (17) gelangt beispielsweise trichterförmig in den Ausatemspalt (14). Der Ausatemspalt ist durch die Strömungsleitfläche (16) begrenzt. Im Ausatemspalt wird der Atemgasstrom zumindest einmal an der Strömungsleitfläche (16) umgelenkt. Entlang der Strömungsleitfläche (16) gelangt der Atemgasstrom vom Inneren der Atemmaske nach außen. Bei der Passage des Atemgasstromes durch den Ausatemspalt (14) entlang der Strömungsleitfläche (16) in die Umgebung wird der Atemgasstrom in einem Winkel Beta (β), der vorzugsweise zwischen 1° und 15° zu einer Vertikalen (38) liegt abgelenkt. Diese Vertikale steht im wesentlichen in einem Winkel von 90° senkrecht zu der Ebene, die durch die den Ausatemspalt begrenzenden Teile des Maskenkörpers gebildet wird. Bei der Passage des Atemgasstromes durch den Ausatemspalt (14) entlang der Strömungsleitfläche (16) in die Umgebung wird der Atemgasstrom besonders bevorzugt in einem Winkel Beta (β), der zwischen 2° und 7° zu einer Vertikalen (38) liegt abgelenkt.

Zur weiteren Erläuterung der Funktionalität der einzelnen Bauelemente wird nunmehr ausgehend von einem demontierten Zustand die auch von einem Patienten in einfacher Weise vornehmbare Montage der Einzelteile erläutert. In einem ersten Schritt werden der aus dem härteren Material bestehende Maskenkörper (1) und der Maskenwulst (2) zusammengefügt. Der Maskenwulst (2) wird hierzu mit einem in den Zeichnungen nicht näher dargestellten U-Profil auf einen Rand des Maskenkörpers (1) aufgeschoben. Im Bereich der Kontaktstelle zwischen Maskenwulst und Maskenkörper befindet sich zumindest ein Hinterschnitt und zumindest ein zum Hinterschnitt komplemantärer Vorsprung. Bevorzugt befindet sich der Hinterschnitt im Bereich des weicheren Bauteiles. Das Zusammenwirken von Hinterschnitt und Vorsprung im montierten Zustand sorgt für eine hohe Verbindungsfestigkeit.

In einem nächsten Montageschritt kann beispielsweise die Stirnstütze (2) mit einem Schaft (35) in eine Halterung (36) des Maskenkörpers (1) eingesetzt und unter Verwendung zumindest eines Befestigungsmittels (37) arretiert werden. Die Montage der Stirnstütze (5) kann aber auch zu beliebigen anderen Zeitpunkten des Montagevorganges erfolgen.

In einem weiteren Montageschritt wird der Sicherungsring (31) ausgehend von der Hülse (4) auf das Anschlußstück (3) aufgeschoben und im Bereich des Kugelgelenkes (18) positioniert. Das Aufschieben des Sicherungsringes (31) erfolgt hierbei in einer Orientierung derart, daß nach einem Abschluß des Aufsetzvorganges die Bajonettzähne (26) in eine der Hülse (4) abgewandte Richtung weisen und das Kugelgelenk (18) vom Kugelkäfig (24) des Sicherungsringes (31) bereichsweise umschlossen ist.

In einem abschließenden Montageschritt wird der Sicherungsring (31) gemeinsam mit dem Anschlußstück (3) im Bereich des Zentrierringes (13) des Maskenkörpers (1) positioniert. Aufgrund der beispielsweise in Fig. 3 zu erkennenden entlang eines Umlaufweges unsymmetrischen Anordnung der Rippen (11) und einer hierzu korrespondierenden Anordnung der Bajonettzähne (26) lassen sich die Bajonettzähne (26) nur in einer einzigen vorgegebenen Positionierung in die Aussparungen (37) zwischen den Rippen (11) einführen. Hierdurch wird eine Codierung bereitgestellt.

Nach dem Einführen der Bajonettzähne (26) in die Aussparungen (37) zwischen den Rippen (11) erfolgt eine Verdrehung des Sicherungsringes (31) relativ zum Maskenkörper (1) derart, daß die Verrastung (12) wirksam wird. Die Verrastung (12) wird vorzugsweise als ein Vorsprung des Sicherungsringes (31) ausgebildet, der in eine korrespondierende Vertiefung des Maskenkörpers (1) eingreift. Grundsätzlich ist aber auch eine umgekehrte Ausbildung denkbar. Ein elastisches Einrasten der Verrastung (12) wird dadurch unterstützt, daß der Sicherungsring (31) aus einem relativ weichen Material ausgebildet ist, so daß der ebenfalls weiche Vorsprung des Sicherungsringes (31) in die Ausnehmung des Maskenkörpers (1) einführbar und auch wieder aus dieser herausdrehbar ist.

Nach einer den Montagevorgang abschließenden Verdrehung des Sicherungsringes (31) relativ zum Maskenkörper (1) ist der Montagevorgang abgeschlossen. Die Endposition des Sicherungsringes (31) ist durch einen seitlichen Anschlag der Bajonettzähne (26) an den Rippen (11) vorgegeben. Die Bajonettzähne (26) hintergreifen darüber hinaus die Rippen (11), so daß die Gesamtanordnung auch Zugbelastungen standhält. Eine Demontage der Atemmaske erfolgt in umgekehrter Reihenfolge wie vorstehend für die Montage beschrieben.

Die Konstruktion des Sicherungsringes (31) führt dazu, daß der Sicherungsring (31) spielfrei und gegebenenfalls unter Vorspannung auf den Maskenkörper (1) aufgesetzt werden kann. Dies hat zur Folge, daß die Weite des oder der Ausatemspalte (14) nur noch von der Toleranz der Höhe der Distanzrippen (25) abhängig ist. Die Realisierung des Ausatemspaltes (14) erfolgt hierdurch äußerst gleichmäßig und weitgehend toleranzunabhängig. Dies wiederum hat zur Folge, daß die Abströmung des Atemgases durch den Ausatemspalt (14) hindurch und die hierdurch hervorgerufenen Schallemissionen äußerst konstant realisiert werden.

Die Konstruktion des Sicherungsringes (31) ermöglicht, daß das Kugelgelenk (18) in den federnden Kugelkäfig (24) des Sicherungsringes (31) eingeführt werden kann und dass der Kugelkäfig (24) das Kugelgelenk zumindest bereichsweise umschließt. Die Fixierung des Kugelgelenks (18) im Kugelkäfig (24) erfolgt dadurch, dass der Zentrierring (13) in die im Sicherungsring befindliche Aufnahme (30) eingeführt wird. Dadurch werden die federnden Elemente des Kugelkäfigs (24) auf der einen Seite durch das Kugelgelenk und auf der gegenüberliegenden Seite durch den zentrierring (13) begrenzt. Im montierten Zustand ist dadurch das Kugelgelenk im Bereich der Maske fixiert. Die Beweglichkeit des Kugelgelenkes einerseits und die Abdichtung gegenüber dem Atemgas im Bereich zwischen Kugelgelenk und Kugelkäfig wird durch die genaue Dimensionierung und enge Toleranzmaße bestimmt.

## Patentansprüche

1. Atemmaske mit einem Maskenkörper und einem gelenkigen Anschlußstück (18), welches mit einem Atemschlauch verbindbar ist, sowie bei der im Bereich des Maskenkörpers (1) mindestens ein Ausatemspalt (14) angeordnet ist, **dadurch gekennzeichnet, daß** für die Verbindung von Maskenkörper (1) und Anschlußstück (18) eine mechanische Kodierung in dem Maskenkörper (1) vorgesehen ist.

2. Atemmaske nach Anspruch 1, **dadurch gekennzeichnet, daß** der Ausatemspalt (14) im Bereich eines das gelenkige Anschlußstück (18) aufnehmenden Anschlusses am Maskenkörper angeordnet ist.

3. Atemmaske nach Anspruch 1 oder 2, **dadurch gekennzeichnet, daß** das gelenkige Anschlußstück (18) als Kugelgelenk ausgebildet ist.

4. Atemmaske nach Anspruch 3, **dadurch gekennzeichnet, daß** das Kugelgelenk (18) an einzelnen Punkten, insbesondere entlang von zwei Führungslinien (33, 34), in einem Kugelkäfig (24) eines aufnehmenden Sicherungsringes (31) aufliegt.

5. Atemmaske nach einem der Ansprüche 1 bis 4, **dadurch gekennzeichnet, daß** einen Ausströmkanal begrenzende Ausströmflächen (10, 28) benachbart zu mindestens einem Distanzelement (25) angeordnet und spielfrei oder mit Vorspannung zueinander versehen sind.

6. Atemmaske nach einem der Ansprüche 4 bis 5, **dadurch gekennzeichnet, daß** der Maskenkörper (1) und das Anschlußstück (18) über den Sicherungsring (31) mit einem Bajonettverschluß (6),(26) gegeneinander fixiert sind.

7. Atemmaske nach Anspruch 5, **dadurch gekennzeichnet, daß** die Ausströmflächen (10, 28) einen Ausströmkanal begrenzen, der der Atemgasstrom (17) im Winkel (α) zwischen 10° und 45° bezogen auf eine Ebene frontal zum Gesicht eines Patienten ableitet.

8. Atemmaske nach einem der Ansprüche 5 oder 7, **dadurch gekennzeichnet, daß** der Ausströmkanal eine Ableitung der Ausatemströmung (17) vorsieht, die ein Segment zur Augenpartie eines Patienten hin ausspart.

9. Atemmaske nach einem der Ansprüche 5 oder 7 bis 8, **dadurch gekennzeichnet, daß** an den Ausströmungskanal grenzende Oberflächen der Atemmaske aus hartem Kunststoff ausgebildet sind und Oberflächen von mit der Hand bedienbaren Elementen aus weichem Kunststoff ausgebildet sind.

10. Atemmaske nach einem der Ansprüche 7 bis 9, **dadurch gekennzeichnet, daß** der winkel (α) in einem Bereich von 20° bis 30° Grad liegt.

11. Atemmaske nach einem der Ansprüche 7 bis 10, **dadurch gekennzeichnet, daß** der Winkel (α) einen Wert von etwa 25° Grad aufweist.

12. Atemmaske nach einem der Ansprüche 4 bis 11, **dadurch gekennzeichnet, daß** im Bereich des Maskenkörpers (1) angeordnete Rippen (11) zur Fixierung des Sicherungsringes (31) entlang eines Umfanges einer Durchgangsausnehmung unsymmetrisch angeordnet sind.

13. Atemmaske nach Anspruch 12, **dadurch gekennzeichnet, daß** Bajonettzähne (26) des Sicherungsringes (31) korrespondierend zu den Rippen (11) entlang eines Umfanges des Sicherungsringes (31) unsymmetrisch angeordnet sind.

14. Atemmaske nach Anspruch 13, **dadurch gekennzeichnet, daß** sich zwischen mindestens zwei Rippen (11) in Umfangsrichtung Aussparungen (37) zur Aufnahme der Bajonettzähne (26) erstrecken.

15. Atemmaske nach einem der Ansprüche 4 bis 14, **dadurch gekennzeichnet, daß** mindestens eine Verrastung (12) zur Arretierung des Sicherungsringes (31) relativ zum Maskenkörper (1) vorgesehen ist.

16. Atemmaske nach einem der Ansprüche 4 bis 15, **dadurch gekennzeichnet, daß** der vom Sicherungsring (31) ausgebildete Kugelkäfig (24) für das Kugelgelenk (18) aus einer Mehrzahl von Einzellamellen ausgebildet ist.

## Claims

1. Respiratory mask with a mask body and an articulated connecting piece (18) which can be connected to a respiratory hose, in which mask at least one exhalation gap (14) is arranged in the region of the mask body (1), **characterised in that** mechanical coding is provided in the mask body (1) for connecting said mask body (1) and the connecting piece (18).

2. Respiratory mask according to claim 1, **characterised in that** the exhalation gap (14) is arranged in the region of a connection on the mask body that receives the articulated connecting piece (18).

3. Respiratory mask according to claim 1 or 2, **characterised in that** the articulated connecting piece (18) is constructed in the form of a ball joint.

4. Respiratory mask according to claim 3, **characterised in that** the ball joint (18) is supported at individual points, particularly along two guide lines (33, 34), in a ball cage (24) on a receiving securing ring (31).

5. Respiratory mask according to one of clams 1 to 4, **characterised in that** outflow faces (10, 28) which delimit an outflow duct are arranged adjacent to at least one distance element (25) and are provided without play or with pretensioning in relation to one another.

6. Respiratory mask according to either of claims 4 or 5, **characterised in that** the mask body (1) and the connecting piece (18) are fixed against one another via the securing ring (31) by means of a bayonet catch (6), (26).

7. Respiratory mask according to claim 5, **characterised in that** the outflow faces (10, 28) delimit an outflow duct which conducts away the respiratory gas flow (17) at an angle (α) of between 10° and 45°, referred to a plane which is frontal in relation to a patient's face.

8. Respiratory mask according to either of claims 5 or 7, **characterised in that** the outflow duct provides for an outlet for the exhalation flow (17) that leaves a segment clear for a patient's eye region.

9. Respiratory mask according to one of claims 5 or 7 to 8, **characterised in that** surfaces of the respiratory mask that border the outflow duct are constructed from hard plastic, and surfaces of elements that can be operated by hand are constructed from soft plastic.

10. Respiratory mask according to one of claims 7 to 9, **characterised in that** the angle (α) lies within a range from 20° to 30°.

11. Respiratory mask according to one of claims 7 to 10, **characterised in that** the angle (α) has a value of about 25°.

12. Respiratory mask according to one of claims 4 to 11, **characterised in that** ribs (11) for fixing the securing ring (31) which are arranged in the region of the mask body (1) are arranged along a periphery of a through-aperture.

13. Respiratory mask according to claim 12, **characterised in that** bayonet teeth (26) on the securing ring (31) are arranged asymmetrically, in a manner corresponding to the ribs (11), along a periphery of said securing ring (31).

14. Respiratory mask according to claim 13, **characterised in that** clearances (37) for receiving the bayonet teeth (26) extend between at least two ribs (11) in the peripheral direction.

15. Respiratory mask according to one of claims 4 to 14, **characterised in that** at least one locating device (12) is provided for locking the securing ring (31) relative to the mask body (1).

16. Respiratory mask according to one of claims 4 to 15, **characterised in that** the ball cage (24) formed for the ball joint (18) by the securing ring (31) is constructed from a plurality of individual lamellae.

## Revendications

1. Masque respiratoire, avec un corps de masque et une pièce de raccordement articulée (18), qui peut être reliée à un tuyau respiratoire, ainsi que dans lequel au moins une fente d'expiration (14) est disposée dans la région du corps de masque (1), **caractérisé en ce qu'**un codage mécanique est prévu dans le corps de masque (1) pour la liaison entre le corps de masque (1) et la pièce de raccordement articulée (18).

2. Masque respiratoire selon la revendication 1, **caractérisé en ce que** la fente d'expiration (14) est disposée dans la région d'un raccord au corps de masque, qui reçoit la pièce de raccordement articulée (18).

3. Masque respiratoire selon revendication 1 ou 2, **caractérisé en ce que** la pièce de raccordement articulée (18) est réalisée sous la forme d'un joint à rotule.

4. Masque respiratoire selon la revendication 3, **caractérisé en ce que** le joint à rotule (18) s'appuie en plusieurs points, en particulier le long de deux lignes de guidage (33, 34), dans une bague de sécurité (31), recevant une cage (24).

5. Masque respiratoire selon l'une des revendications 1 à 4, **caractérisé en ce que** des surfaces de sortie (10, 28), qui limitent un canal d'écoulement, sont disposées dans le voisinage d'au moins un élément d'écartement (25) et sont prévues sans jeu, ou sous précontrainte l'une par rapport à l'autre.

6. Masque respiratoire selon l'une des revendications 4 à 5, **caractérisé en ce que** le corps de masque (1) et la pièce de raccordement articulée (18) sont fixés l'un par rapport à l'autre avec une fermeture à baïonnette (6, 26), par l'intermédiaire de la bague de sécurité (31).

7. Masque respiratoire selon la revendication 5, **caractérisé en ce que** les surfaces d'écoulement (10, 28) limitent un canal d'écoulement, qui dévie le flux de gaz respiratoire (17) sous un angle (α), situé entre 10 ° et 45 ° avec un plan frontal par rapport au visage d'un patient.

8. Masque respiratoire selon l'une des revendications 5 ou 7, **caractérisé en ce que** le canal d'écoulement prévoit une dérivation du flux d'air expiré (17), qui dégage un segment en direction des yeux d'un patient.

9. Masque respiratoire selon l'une des revendications 5 ou 7 à 8, **caractérisé en ce que** des surfaces du masque respiratoire, adjacentes au canal d'écoulement, consistent en matière plastique dure et que des surfaces d'éléments, actionnés à la main, sont en matière plastique souple.

10. Masque respiratoire selon l'une des revendications 7 à 9, **caractérisé en ce que** l'angle (α) est situé dans une plage allant de 20 ° à 30 °.

11. Masque respiratoire selon l'une des revendications 7 à 10, **caractérisé en ce que** l'angle (α) présente une valeur d'environ 25 °.

12. Masque respiratoire selon l'une des revendications 4 à 11, **caractérisé en ce que**, dans la région du corps de masque (1), sont prévues des nervures (11), qui, destinées à la fixation de la bague de sécurité (31), sont disposées asymétriquement le long d'un pourtour d'un passage.

13. Masque respiratoire selon la revendication 12, **caractérisé en ce que** les dents de la fermeture à baïonnette (26) de la bague de sécurité (31) sont disposées asymétriquement, en concordance avec les nervures (11), le long d'un pourtour de ladite bague de sécurité (31).

14. Masque respiratoire selon la revendication 13, **caractérisé en ce que** des évidements (37), destinées à recevoir les dents de la fermeture à baïonnette (26), s'étendent entre au moins deux nervures (11), dans la direction circonférentielle.

15. Masque respiratoire selon l'une des revendications 4 à 14, **caractérisé en ce qu'**au moins un enclenchement (12) est prévu pour le blocage de la bague de sécurité (31) par rapport au corps de masque (1).

16. Masque respiratoire selon l'une des revendications 4 à 15, **caractérisé en ce que** la cage (24), formée dans la bague de sécurité (31) pour le joint à rotule (18), est constituée par une pluralité de lamelles individuelles.
